# EUROPÄISCHE PATENTSCHRIFT

(11) **EP 3 080 070 B1**
(45) Veröffentlichungstag und Bekanntmachung des Hinweises auf die Patenterteilung: **01.11.2017**
(21) Anmeldenummer: 14808633.3
(22) Anmeldetag: 08.12.2014
(51) Int. Cl.: C07C 209/04, C07C 209/08, C07C 211/29, C07C 209/14

(54) **VERFAHREN ZUR HERSTELLUNG VON HALOGENIERTEN DI-SUBSTITUIERTEN BENZYLAMINEN, INSBESONDERE HALOGENIERTEN DIALKYLBENZYLAMINEN**
METHOD FOR THE PREPARATION OF HALOGENATED DI-SUBSTITUTED BENZYLAMINES, IN PARTICULAR HALOGENETED DIALKYLBENZYLAMINES
PROCÉDÉ DE PRÉPARATION DE BENZYLAMINES DISUBSTITUÉES HALOGÉNÉES, EN PARTICULIER DE DIALKYLBENZYLAMINES HALOGÉNÉES

(30) Priorität: 11.12.2013 EP 13196718
(43) Veröffentlichungstag der Anmeldung: 19.10.2016
(73) Patentinhaber: Bayer CropScience Aktiengesellschaft, 40789 Monheim am Rhein (DE)
(72) Erfinder: SEEBA, Johann, 51375 Leverkusen (DE); SCHLEGEL, Günter, 51381 Leverkusen (DE); LITTMANN, Martin, 51375 Leverkusen (DE); WARSITZ, Rafael, 45136 Essen (DE); FORD, Mark James, 65207 Wiesbaden-Breckenheim (DE)
(74) Vertreter: BIP Patents
(86) Internationale Anmeldenummer: PCT/EP2014/076822
(87) Internationale Veröffentlichungsnummer: WO 2015/086490

(56) Entgegenhaltungen:
- WO-A1-2008/125592
- WO-A1-2013/017611

## Beschreibung

Die vorliegende Erfindung betrifft ein neues Verfahren zur Herstellung von halogenierten Di-substituierten Benzylaminen, insbesondere halogenierten Dialkylbenzylaminen.

Aus dem Stand der Technik ist die Herstellung von halogenierten Dialkybenzylaminen bekannt. In der WO 2012/139561 A1 wird beispielsweise ein Verfahren zur Herstellung aromatischer oder heteroaromatischer Amine beschrieben, hergestellt aus den entsprechenden Aryl- und Heteroarylhalogeniden oder Sulfonaten in Gegenwart eines Katalysators und einer Base.

In der US 2007/0073086 A1 wird ein Verfahren zur Herstellung von Diphenylaminen beschrieben, wobei Arylhalogenide mit aromatischen Aminen in Gegenwart eines organischen Lösungsmittels und Alkalihydroxid sowie einem Phasentransferkatalysators umgesetzt werden.

Auch in der US 3,646,147 P wird bereits ein Verfahren zur Herstellung tertiärer Amine offenbart, in welchem Alkylchloride mit primären Aminen in Gegenwart von Alkalimetalliodiden als Katalysatoren umgesetzt werden.

Ein Verfahren zur Herstellung von o-Chloromethylphenylglyoxylester wird ebenfalls in der WO 2008/125592 A1 offenbart. Dort wird die Synthese von 2-Chlorobenzylmorpholin beschrieben, wobei zu einer Lösung aus 2-Chlorobenzylchlorid in Toluol und 15%iger Natronlauge das Morpholin zugegeben wird.

EP 0 034 425 A1 beschreibt ein Verfahren zur Herstellung tertiärer Amine, in welchem ausgehend von einem mono- oder di-sekundären Amin, dieses durch eine Ullman Kondensation mit einer Di-Iodarylverbindung in Gegenwart eines Kupfer-Katalysators aryliert wird.

In der WO 2013/017611 A1 wird ebenfalls ein Verfahren zur Herstellung von N,N-Dialkylbenzylaminen beschrieben. In diesem Verfahren wird Dimethylamin und 2-Chlor-benzylchlorid in einem Molverhältnis von 3 : 1 oder größer umgesetzt.

Nachteilig an den im Stand der Technik beschriebenen Verfahren ist der Einsatz von kostenintensiven Katalysatoren oder die Verwendung eines hohen Überschusses an sekundären Aminen, die eine aufwändige Abtrennung des Amins erforderlich macht.

Aufgabe der vorliegenden Erfindung bestand daher in der Bereitstellung eines neuen Verfahrens zur Herstellung von halogenierten Di-substituierten Benzylaminen, insbesondere halogenierten Dialkylbenzylaminen, in welchem keine Katalysatoren eingesetzt werden müssen und das sekundäre Amin nicht im Überschuss in der Reaktionsmischung vorliegt und dabei gleichzeitig das Endprodukt in hoher Reinheit und Ausbeute erhalten werden kann.

Überraschenderweise wurde nun gefunden, dass mit dem erfindungsgemäßen Verfahren trotz eines geringeren Molverhältnisses zwischen den Benzylhalogenid und Di-substituierten Amin, insbesondere Dialkylamin Ausbeuten von größer gleich 95 % und eine Reinheit des Endproduktes von 99 % erreicht werden kann. Des Weiteren kann hierbei auf die Wiedergewinnung des Amins verzichtet werden.

Gegenstand der vorliegenden Erfindung ist ein Verfahren zur Herstellung von Verbindungen der Formel (I) 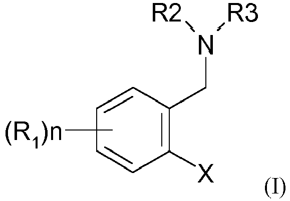 in welcher
- R₁: für Wasserstoff, C₁-C₈-Aklyl, C₂-C₆-Alkenyl, C₃-C₆-Cycloalkyl, C₁-C₈-Alkoxy, C₁-C₆-Alkoxy-C₋₂-C₆-Alkenyl, Phenyl, Benzyl steht, bevorzugt für Wasserstoff steht;
- R₂, R₃: unabhängig voneinander für C₁-C₈-Alkyl, Aryl, Heteroaryl, C₂-C₆-Alkenyl, C₃-C₈-Cycloalkyl, C₁-C₆-Alkoxy, C₁-C₆-Halogenalkyl, C₁-C₆-Halogenalkoxy stehen, bevorzugt für C₁-C₆-Alkyl, besonders bevorzugt für Methyl, Ethyl, n-Propyl, n-Butyl, ganz besonders bevorzugt für Methyl oder Ethyl stehen,
- X: für Chlor oder Brom steht, bevorzugt für Chlor steht und
- n: 0, 1, 2, 3 oder 4 ist, bevorzugt 0 ist;
dadurch gekennzeichnet, dass zunächst als Reaktionsmedium ein Alkalihydroxid in einer Konzentration von 20 bis 50 Gew.-%, bevorzugt von 25 bis 45 Gew.-%, besonders bevorzugt von 30 bis 40 Gew.-% vorgelegt wird, anschließend in einem ersten Schritt A) die Verbindungen (II) und (III) 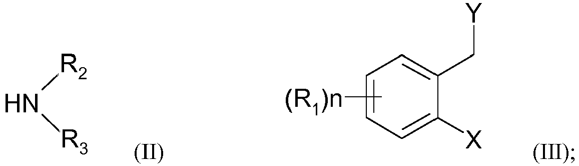 in welchen
- R₁, R₂, R₃: sowie X die oben genannte Bedeutung haben und
- Y: für Chlor, Brom, Iod, Alkylsulfonate (-OSO₂-Alkyl, bevorzugt -OSO₂CH₃, -OSO₂CF₃) oder Arylsulfonate (-OSO₂-Aryl, bevorzugt -OSO₂Ph, -OSO₂PhMe) steht, bevorzugt für Chlor oder Brom, besonders bevorzugt für Chlor steht;

in einem Molverhältnis von 0,9 bis 2,5, bevorzugt 1 bis 2, besonders bevorzugt 1,1 bis 1,3 umgesetzt werden und in einem zweiten Schritt B) 0 bis 9 mol Wasser, bevorzugt 1 bis 5 mol, besonders bevorzugt 1 bis 3 mol, bezogen auf 1 mol der Verbindung der Formel (III) der Reaktionsmischung zugesetzt wird, und anschließend die Abtrennung der wässrigen Phase vom Produkt erfolgt.

Insgesamt ist es bevorzugt, den Schritt A) unter Ausschluss eines organischen Lösungsmittels durchzuführen. Besonders bevorzugt werden die Schritte A) und B) des erfindungsgemäßen Verfahrens beide unter Ausschluss eines organischen Lösungsmittels durchzuführen.

Die Reaktionstemperatur des erfindungsgemäßen Verfahrens liegt zwischen -20°C bis 70 °C, bevorzugt zwischen 0°C bis 65°C, besonders bevorzugt zwischen 20°C bis 60°C.

Das erfindungsgemäße Verfahren kann bei einem Reaktionsdruck von 0,1 bar bis 32 bar, bevorzugt bei 0,5 bis 10 bar, besonders bevorzugt bei 0,9 bar bis 1,5 bar durchgeführt werden.

Ganz besonders bevorzugt als Verbindung der Formel (I) ist 2-Chlor-N,N-dimethylbenzylamin.

Ganz besonders bevorzugt als Verbindung der Formel (II) ist Dimethylamin, welches bevorzugt als 35 Gew.-%ige bis 45 Gew.-%ige, besonders bevorzugt als 40 Gew.-%ige wässrige Lösung eingesetzt wird.

Ganz besonders bevorzugt als Verbindung der Formel (III) ist 2-Chlor-benzylchlorid.

Geeignete Alkalihydroxide sind Kaliumhydroxid oder Natriumhydroxid, bevorzugt ist Natriumhydroxid.

Bevorzugt werden die Verbindungen der Formel (II) und (III) in Schritt A) des erfindungsgemäßen Verfahrens innerhalb von 0,2 bis 6 h der vorgelegten Natronlauge zugegeben. Ebenfalls möglich und ebenfalls bevorzugt ist es, zuerst die Verbindungen der Formel (II) der Natronlauge zuzugeben und anschließend Verbindungen der Formel (III) aufzudosieren.

Gegebenenfalls kann in Schritt A) auch Verbindung der Formel (III) und Natronlauge vorgelegt werden und die Verbindung der Formel (II) zugegeben werden.

Bevorzugt schließt sich nach der vollständigen Dosierung der Verbindungen der Formel (II) und (III) in Schritt A) des erfindungsgemäßen Verfahrens eine Nachreaktionszeit von 1 bis 24 h, bevorzugt 4 bis 18 h, besonders bevorzugt von 6 bis 12 h an.

Gegebenenfalls kann sich nach Zugabe des Wassers in Schritt B) des erfindungsgemäßen Verfahrens ein Reinigungsschritt C) anschließen. Dazu wird der Reaktionsmischung ein organisches Lösungsmittel im

Molverhältnis Lösemittel zu Rohprodukt von 1 : 1 bis 1 : 50, bevorzugt von 1 : 5 bis 1 : 20, besonders bevorzugt von 1 : 5 bis 1 : 10 zugegeben. Besonders bevorzugt wird dabei das Reaktionsmedium auf einen pH-Wert (RT) zwischen 0 und 2 eingestellt, indem Mineralsäure, bevorzugt Salzsäure oder Schwefelsäure, besonders bevorzugt Salzsäure zugegeben wird. Es folgt die Abtrennung der organischen Phase, wobei die wässrige Phase mit einem organischen Lösungsmittel versetzt wird und das Molverhältnis zwischen Lösemittel zu Rohprodukt von 10 : 1 bis 1 : 50, bevorzugt von 5: 1 bis 1 : 10, besonders bevorzugt von 2 : 1 bis 1 : 2 beträgt. Dabei wird der pH-Wert (RT) zwischen 11 und 14 eingestellt, indem eine Base, bevorzugt Natronlauge zugegeben wird. Anschließend wird die wässrige Phase abgetrennt.

In einer bevorzugten Ausführungsform des erfindungsgemäßen Verfahrens ist der Reinigungsschritt C) enthalten.

In einem weiteren Schritt D) wird das organische Lösungsmittel vom Produkt abgetrennt, bevorzugt mittels Destillation. Falls auf Schritt C) verzichtet wurde, kann für Schritt D) ein organisches Lösungsmittel eingesetzt werden, das bei der Destillation wieder entfernt wird. Im erfindungsgemäßen Verfahren dient die Destillation ausschließlich der Entfernung von Wasserresten und gegebenenfalls von Resten des organischen Lösungsmittels sowie von Resten der Verbindung der Formel (II). Die Verbindung (I) selber wird nicht abdestilliert. Sie wird nach Durchführung von Schritt D) in hoher Reinheit erhalten.

Bevorzugt enthält das erfindungsgemäße Verfahren die Schritte A), B) und D). Besonders bevorzugt enthält es die Schritte A), B), C) und D).

Als organische Lösungsmittel kommen unpolare Lösemittel in Frage wie z.B. Toluol, Methylcyclohexan oder Methyl-tert.-Butylether in Frage. Bevorzugt ist Toluol.

Das erfindungsgemäße Verfahren kann sowohl batchweise als auch kontinuierlich durchgeführt werden, wobei die batchweise Verfahrensweise bevorzugt ist.

Bei der kontinuierlichen Fahrweise kann beispielsweise für den Schritt A) ein Rohrreaktor eingesetzt werden. Erfindungsgemäß wird Natriumhydroxid kontinuierlich durch das Rohr geströmt und dann die Verbindung der Formel (II) z.B. mittels Düse oder Statischen Mischer zugemischt. Anschließend erfolgt die Dosierung stromabwärts der Verbindung der Formel (III). Es ist auch möglich die Zugabe an mehreren Stellen des Reaktors in Teilströmen vorzunehmen. Um eine gute Durchmischung der Phasen zu erreichen, muss dabei eine ausreichende Strömungsgeschwindigkeit im Rohrreaktor eingestellt werden.

Anschließend erfolgt der Schritt B) wie beim diskontinuierlichen Prozess oder ebenfalls in einem kontinuierlichen Prozess. Bei dem kontinuierlichen Prozess für Schritt B) wird das Wasser am Ende des Rohrreaktors kontinuierlich zugegeben. Gegebenenfalls folgt hier ein weiterer Rohrreaktor. Die Phasentrennung kann beispielweise mit einem kontinuierlichen Phasenscheider durchgeführt werden. Alternativ kann die Wasserzugabe und Phasentrennung in einer Mixer-Settler-Apparatur erfolgen.

Anschließend kann der Schritt C) durchgeführt werden. Dies kann erneut diskontinuierlich erfolgen, oder mit mehreren Mixer-Settler-Apparaturen kontinuierlich durchgeführt werden.

Danach kann der Schritt D) erfolgen. Auch hier kann die Destillation entweder diskontinuierlich durchgeführt werden oder in Form einer kontinuierlichen Destillation erfolgen.

Ebenfalls möglich und bevorzugt ist die kontinuierliche Fahrweise des erfindungsgemäßen Verfahrens in einer Rührkesselkaskade. Dabei wird die Natronlauge und die Verbindung der Formel (II) in einem Rührkessel vermischt und das Gemisch kontinuierlich in einen zweiten Rührkessel überführt. Dort wird die Verbindung der Formel (III) kontinuierlich zugemischt. Dieses Gemisch wird anschließend durch weitere Rührkessel gefördert, um die Reaktion zu vervollständigen. Die Zahl der aufeinander folgenden Rührkessel kann 1 bis n betragen, mit n gleich 20. Bevorzugt beträgt n 1 bis 10, besonders bevorzugt 1 bis 3. Es können auch die Natronlauge und die Verbindungen der Formel (II) und der Formel (III) gemeinsam im ersten Rührkessel kontinuierlich mit der Natronlauge vermischt werden und anschließend in 1 bis n weitere Rührkessel überführt werden.

Anschließend erfolgt die Aufarbeitung im Schritt B) wie oben beschrieben diskontinuierlich oder kontinuierlich. Es können wieder die Schritte C) und D) oder einer von beiden Schritten durchgeführt werden. Diese Schritte können wie oben beschrieben kontinuierlich oder diskontinuierlich durchgeführt werden.

Aufgrund der hohen Reinheit der durch das erfindungsgemäße Verfahren erhältlichen Verbindungen der Formel (I) lassen diese sich ohne weiteren Reinigungsschritt direkt in Metallisierungsreaktionen wie beispielsweise Grignard-Reaktionen einsetzen.

Bei den in den vorstehenden Formeln angegebenen Definitionen der Symbole wurden Sammelbegriffe verwendet, die allgemein repräsentativ für die folgenden Substituenten stehen:
**Alkyl:** gesättigte, geradkettige oder verzweigte Kohlenwasserstoffreste mit 1 bis 8 vorzugsweise 1 bis 6 und noch bevorzugter 1 bis 3 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Diese Definition gilt auch für Alkyl als Bestandteil eines zusammengesetzen Substitutenten wie z.B. Cycloalkylalkyl, Hydroxyalkyl etc. sofern an anderer Stelle nicht definiert wie z.B. Alkylthio, Alkylsufinyl, Alkylsulfonyl, Halogenalkyl bzw. Halogenalkylthio. Steht das Alkyl am Ende eines zusammengesetzten Substituenten. wie z.B bei Alkylcylcloalkyl, so kann der am Anfang stehende Bestandteil des zusammengesetzten Substituenten, z.B. das Cycloalkyl, ein- bzw. mehrfach, gleich oder verschieden und unabhängig voneinander mit Alkyl substituiert sein. Das gleiche gilt auch für zusammengesetzte Substituenten bei denen andere Reste wie z.B. Alkenyl, Alkinyl, Hydroxy, Halogen, Formyl etc. am Ende stehen.
**Alkenyl:** ungesättigte, geradkettige oder verzweigte Kohlenwasserstoffreste mit 2 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl. Diese Definition gilt auch für Alkenyl als Bestandteil eines zusammengesetzen Substitutenten wie z.B. Halogenalkenyl etc. sofern an anderer Stelle nicht definiert;
**Alkoxy:** gesättigte, geradkettige oder verzweigte Alkyloxy-, Alkenyloxy- oder Alkyinyloxyreste mit 1 6 und bevorzugt 1 bis 3 Kohlenstoffatomen, haben, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy oder C₁-C₆-Alkenyloxy wie But-3-en-1-yloxy und Allyloxy oder C₁-C₆-Alkinyloxy wie Prop-2-in-1-yloxy, But-2-in-1-yloxy, Pent-2-in-1-yloxy, But-3-in-1-yloxy. Diese Definition gilt auch für Alkoxy als Bestandteil eines zusammengesetzen Substitutenten wie z.B. Halogenalkoxy, Alkinylalkoxy etc. sofern an anderer Stelle nicht definiert;
**Cycloalkyl:** monocyclische, gesättigte Kohlenwasserstoffgruppen mit 3 bis 8 und bevorzugt 3 bis 6 Kohlenstoffringgliedern, z.B. (aber nicht beschränkt auf) Cyclopropyl, Cyclopentyl und Cyclohexyl. Diese Definition gilt auch für Cycloalkyl als Bestandteil eines zusammengesetzen Substitutenten wie z.B. Cycloalkylalkyl etc. sofern an anderer Stelle nicht definiert;
**Halogenalkyl:** geradkettige oder verzweigte Alkylgruppen mit 1 bis 6 und bevorzugtl bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl. Diese Definition gilt auch für Halogenalkyl als Bestandteil eines zusammengesetzen Substitutenten wie z.B. Halogenalkylaminoalkyl etc. sofern an anderer Stelle nicht definiert;
**Halogenalkoxy:** geradkettige oder verzweigte Alkoxygruppen mit 1 bis 6 und bevorzugt 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy. Diese Definition gilt auch für Halogenalkoxy als Bestandteil eines zusammengesetzen Substitutenten wie z.B. Halogenalkoxyalkyl etc. sofern an anderer Stelle nicht definiert;
**Aryl:** Phenyl oder Naphthyl, bevorzugt Phenyl.

Das erfindungsgemäße Verfahren wird durch folgende Beispiele veranschaulicht, ohne auf sie eingeschränkt zu sein.

Im Folgenden soll unter der Abkürzung "Eq" die Molequivalente verstanden werden. Als Molequivalente wird die Molzahl der jeweiligen Komponenten dividiert durch die Molzahl an 2-Chlorbenzylchlorid bezeichnet. 2-Chlorbenzylchlorid liegt somit immer definitionsgemäß mit 1,0 Molequivalenten vor.

### Beispiel 1 (erfindungsgemäßes Verfahren)

### Schritt A) und Schritt B):

405,1 g 32%ige Natronlauge (1,07 Eq) wurden vorgelegt und parallel 469,3g 35%ige Lösung von Dimethylamin (1,2 Eq) in Wasser und gleichzeitig 498,7 g 2-Chlorbenzylchlorid (1,0 Eq) in einer Stunde bei 40°C zudosiert. Es wurde 6 Stunden bei 40°C nachgerührt. Nach Zugabe von 85g Wasser wurden die beiden flüssigen Phasen getrennt.

Es wurden 512,2 g organische Phase erhalten. Gehalt der Verbindung der Formel (I) 99,3%. Ausbeute 98,7%.

### Schritt C)

512,2 g der organischen Phase wurden mit 84,1g Toluol und 557,9 g 20%iger Salzsäure bei 30°C verrührt (pH 1) und anschließend wurden die Phasen getrennt. Die organische Phase wurdr verworfen. Die wässrige Phase wurde mit 603 g Toluol und 599,6g 20%iger Natronlauge versetzt (pH 12). Die Phasen wurden getrennt.

Es wurden 1104,6g organische Phase erhalten. Ausbeute 99%.

### Schritt D)

1104,6 g organische Phase wurden bei reduziertem Druck destilliert. Es wurden 502,9 g Rückstand erhalten. Reinheit der Verbindung der Formel (I) 99% (HPLC), Ausbeute 96,7%.

### Beispiel 2

### Verfahren mit den Schritten A),B) und D) (d.h. keine Reinigung nach Schritt C)

### Schritt A) und B)

3996 g 32%ige Natronlauge (1,05 Eq) wurden bei -10°C vorgelegt und parallel 4125 g 40%ige Lösung von Dimethylamin (1,2 Eq) in Wasser und gleichzeitig 5005g 2-Chlorbenzylchlorid (1,0 Eq) in 70 Minuten zudosiert. Die Temperatur stieg während der Dosierung auf +30°C an. Es wurde 12 Stunden bei 40°C nachgerührt. Nach Zugabe von 850g Wasser wurden die beiden flüssigen Phasen getrennt.

Es wurden 5129,6 g organische Phase erhalten. Gehalt der Verbindung der Formel (I) 99,2% (HPLC). Ausbeute 98,4%.

### Schritt D)

5129,6 g organische Phase wurden mit 849 g Toluol gemischt und bei reduziertem Druck destilliert. Es wurden 5086 g Rückstand erhalten. Reinheit der Verbindung der Formel (I) 98,1% (HPLC), Ausbeute 96,6%.

### Beispiel 3

### Verfahren aus WO 2008/125592 A1mit Morpholin

200g 2-Chlorbenzylchlorid (1,0 Eq) wurden vorgelegt und mit 346g Toluol gemischt. Es wurden 422g 15%ige Natronlauge (1,3 Eq) zugetropft und anschließend 128,5g Morpholin (1,2 Eq) zudosiert. Die Mischung wurde zum Rückfluss erhitzt und über Nacht bei Rückfluss gerührt. Dann wurden die Phasen getrennt und die organische Phase mit 200g Wasser gewaschen. Die gewaschene organische Phase wurde durch azeotrope Destillation getrocknet und das Lösemittel im Vakuum teilweise abdestilliert. Es wurden 271g 2-(Morpholinomethyl)-chlorbenzol erhalten. Reinheit der Verbindung: 90,9% (HPLC). Ausbeute: 95,6%.

### Beispiel 4

Verfahren aus WO 2008/125592 A1mit Morpholin ohne Zusatz eines organischen Verdünnungsmittels

32,9g 2-Chlorbenzylchlorid (1,0 Eq) wurden vorgelegt. Es wurden 69,3g 15%ige Natronlauge (1,3 Eq) zugetropft und anschließend 21,1g Morpholin (1,2 Eq) zudosiert. Die Mischung wurde zum Rückfluss erhitzt und über Nacht bei Rückfluss gerührt. Dann wurden die Phasen getrennt und die organische Phase mit 32,2g Wasser gewaschen. Die gewaschene organische Phase wurde durch Destillation im Vakuum getrocknet. Es wurden 36,5g 2-(Morpholinomethyl)-chlorbenzol erhalten. Reinheit der Verbindung: 97,8% (HPLC), Ausbeute: 84,3%

### Beispiel 5

### Verfahren aus WO 2008/125592 A1 angewendet auf Dimethylamin

32,9g 2-Chlorbenzylchlorid (1,0 Eq) wurden vorgelegt und mit 55,7g Toluol verdünnt. Es wurden 23,1g 45%ige Natronlauge (1,3 Eq) mit 30g Wasser verdünnt und zu der Mischung aus 2-Chlorbenzylchlorid und Toluol zugetropft. Anschließend wurden 27,1g einer 40%igen wässrige Lösung von Dimethylamin (1,2 Eq) zudosiert. Unter Berücksichtigung des Wassers aus dem Dimethylamin ergibt sich eine 15%ige Natronlauge. Die Mischung wurde zum Rückfluss erhitzt und über Nacht bei Rückfluss gerührt. Dann wurden die Phasen getrennt und die organische Phase mit 32,2g Wasser gewaschen. Die gewaschene organische Phase wurde durch azeotrope Destillation im Vakuum getrocknet. Es wurden 33,6g eines gelben Öls erhalten. Reinheit der Verbindung der Formel (I): 81,3% (HPLC), Ausbeute: 80,5%

## Patentansprüche

1. Verfahren zur Herstellung von Verbindungen der Formel (I) 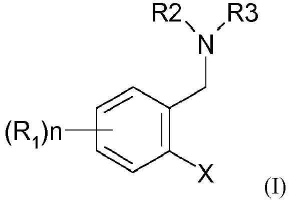 in welcher
R₁ für Wasserstoff, C₁-C₈-Alkyl, C₂-C₆-Alkenyl, C₃-C₆-Cycloalkyl, C₁-C₈-Alkoxy, C₁-C₆-Alkoxy-C₂-C₆-Alkenyl, Phenyl, Benzyl steht;
R₂, R₃ unabhängig voneinander für C₁-C₈-Alkyl, Aryl, Heteroaryl, C₂-C₆-Alkenyl, C₃-C₈-Cycloalkyl, C₁-C₆-Alkoxy, C₁-C₆-Halogenalkyl, C₁-C₆-Halogenalkoxy stehen;
X für Chlor oder Brom steht und
n 0, 1, 2, 3 oder 4 ist;
**dadurch gekennzeichnet, dass** zunächst als Reaktionsmedium ein Alkalihydroxid in einer Konzentration von 20 bis 50 Gew.-% vorgelegt wird, anschließend in einem ersten Schritt A) die Verbindungen (II) und (III) 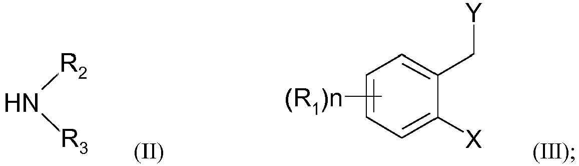 in welchen
R₁, R₂, R₃ sowie X die oben genannte Bedeutung haben und
Y für Chlor, Brom, Iod, Alkylsulfonate (-OSO₂-Alkyl, bevorzugt -OSO₂CH₃, -OSO₂CF₃) oder Arylsulfonate (-OSO₂-Aryl) steht;
in einem Molverhältnis von 0,9 bis 2,5 umgesetzt werden und in einem zweiten Schritt B) 0 bis 9 mol Wasser, bezogen auf 1 mol der Verbindung der Formel (III) der Reaktionsmischung zugesetzt wird, und anschließend die Abtrennung der wässrigen Phase vom Produkt erfolgt.

2. Verfahren gemäß Anspruch 1, **dadurch gekennzeichnet, dass** der Schritt A) unter Ausschluss eines organischen Lösungsmittels durchgeführt wird.

3. Verfahren gemäß Anspruch 1, **dadurch gekennzeichnet, dass** die Schritte A) und B) unter Ausschluss eines organischen Lösungsmittels durchgeführt werden.

4. Verfahren gemäß Anspruch 1, **dadurch gekennzeichnet, dass** die Reaktionstemperatur zwischen - 20°C bis 70 °C liegt.

5. Verfahren gemäß Anspruch 1, **dadurch gekennzeichnet, dass** das Alkalihydroxid Natriumhydroxid ist.

6. Verfahren gemäß Anspruch 1, **dadurch gekennzeichnet, dass** sich nach der vollständigen Dosierung der Verbindungen der Formel (II) und (III) in Schritt A) eine Nachreaktionszeit von 1 bis 24 h anschließt.

7. Verfahren gemäß Anspruch 1, **dadurch gekennzeichnet, dass** sich an Schritt B) ein Reinigungsschritt C) anschließt, wobei der Reaktionsmischung ein organisches Lösungsmittel im Molverhältnis Lösemittel zu Rohprodukt von 1 : 1 bis 1 : 50 zugegeben wird.

8. Verfahren gemäß Anspruch 7, **dadurch gekennzeichnet, dass** das Reaktionsmedium auf einen pH-Wert (RT) zwischen 0 und 2 eingestellt wird, indem Mineralsäure zugegeben wird.

9. Verfahren gemäß Anspruch 7 und 8, **dadurch gekennzeichnet, dass** die Abtrennung der organischen Phase erfolgt, wobei die wässrige Phase mit einem organischen Lösungsmittel versetzt wird und das Molverhältnis zwischen Lösemittel zu Rohprodukt von 10 : 1 bis 1 : 50 beträgt und der pH-Wert (RT) zwischen 11 und 14 mit einer Base eingestellt wird und die wässrige Phase abgetrennt wird.

10. Verfahren gemäß Anspruch 1 oder 7, **dadurch gekennzeichnet, dass** in einem Schritt D) die Entfernung von Wasserresten und gegebenenfalls von Resten des organischen Lösungsmittels sowie von Resten der Verbindung der Formel (II) durch Destillation erfolgt.

11. Verfahren gemäß Anspruch 1, **dadurch gekennzeichnet, dass** es die Schritte A), B) und D) umfasst.

12. Verfahren gemäß Anspruch 1, **dadurch gekennzeichnet, dass** es die Schritte A), B), C) und D) umfasst.

## Claims

1. Process for preparing compounds of formula (I) 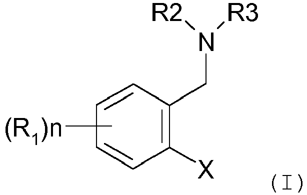 where
R₁ is hydrogen, C₁-C₈-alkyl, C₂-C₆-alkenyl, C₃-C₆-cycloalkyl, C₁-C₈-alkoxy, C₁-C₆-alkoxy-C₂-C₆-alkenyl, phenyl, benzyl;
R₂, R₃ are each independently C₁-C₈-alkyl, aryl, heteroaryl, C₂-C₆-alkenyl, C₃-C₈-cycloalkyl, C₁-C₆-alkoxy, C₁-C₆-haloalkyl, C₁-C₆-haloalkoxy;
X is chlorine or bromine, and
n is 0, 1, 2, 3 or 4;
**characterized in that** it comprises initially charging as reaction medium an alkali metal hydroxide in a concentration of from 20% to 50% by weight, subsequently reacting in a first step A) the compounds (II) and (III) 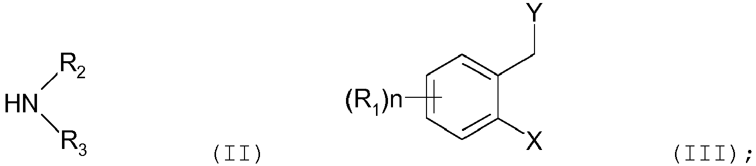 where
R₁, R₂, R₃ and X are as defined above and
Y is chlorine, bromine, iodine, an alkylsulphonate (-OSO₂-alkyl, preferably - OSO₂CH₃, -OSO₂CF₃) or an arylsulphonate (-OSO₂-aryl) ;
in a molar ratio of from 0.9 to 2.5 and adding to the reaction mixture in a second step B) from 0 to 9 mol of water based on 1 mol of the compound of formula (III), and subsequently removing the aqueous phase from the product.

2. Process according to Claim 1, **characterized in that** step A) is carried out in the absence of an organic solvent.

3. Process according to Claim 1, **characterized in that** steps A) and B) are carried out in the absence of an organic solvent.

4. Process according to Claim 1, **characterized in that** the reaction temperature is between -20°C and 70°C.

5. Process according to Claim 1, **characterized in that** the alkali metal hydroxide is sodium hydroxide.

6. Process according to Claim 1, **characterized in that** on completion of the addition of the compounds of formulae (II) and (III) in step A) the mixture is post-reacted for 1 to 24 h.

7. Process according to Claim 1, **characterized in that** step B) is followed by a purification step C), wherein an organic solvent is added to the reaction mixture in a molar ratio of solvent to crude product of from 1 : 1 to 1 : 50.

8. Process according to Claim 7, **characterized in that** the pH of the reaction medium is adjusted to a value (RT) between 0 and 2 by addition of mineral acid.

9. Process according to either of Claims 7 and 8, **characterized in that** it comprises removing the organic phase, wherein the aqueous phase is admixed with an organic solvent and the molar ratio between solvent and crude product is from 10: 1 to 1: 50 and the pH is adjusted to a value (RT) between 11 and 14 with a base and the aqueous phase is removed.

10. Process according to either of Claims 1 and 7, **characterized in that** it comprises distillative removal in a step D) of residual water, any residual amounts of the organic solvent and residual amounts of the compound of formula (II).

11. Process according to Claim 1, **characterized in that** it comprises the steps A), B) and D).

12. Process according to Claim 1, **characterized in that** it comprises the steps A), B), C) and D).

## Revendications

1. Procédé de fabrication de composés de formule (I) 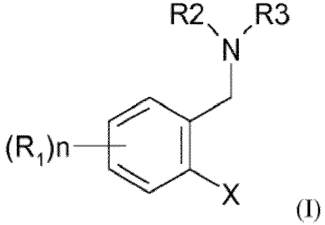 dans laquelle
R₁ représente hydrogène, alkyle en C₁-C₈, alcényle en C₂-C₆, cycloalkyle en C₃-C₆, alcoxy en C₁-C₈, alcoxy en C₁-C₆-alcényle en C₂-C₆, phényle, benzyle ;
R₂, R₃ représente indépendamment l'un de l'autre alkyle en C₁-C₈, aryle, hétéroaryle, alcényle en C₂-C₆, cycloalkyle en C₃-C₈, alcoxy en C₁-C₆, halogénoalkyle en C₁-C₆, halogénoalcoxy en C₁-C₆ ;
X représente chlore ou brome, et
n vaut 0, 1, 2, 3 ou 4 ;
**caractérisé en ce qu'**un hydroxyde alcalin est tout d'abord chargé en tant que milieu de réaction en une concentration de 20 à 50 % en poids, puis, lors d'une première étape A), les composés (II) et (III) 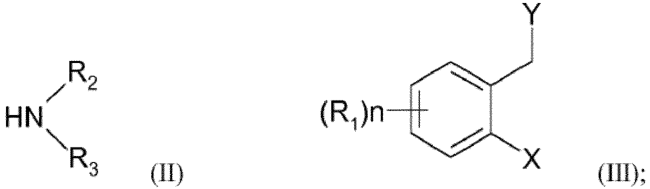 dans lesquels
R₁, R₂, R₃ et X ont la signification indiquée précédemment et
Y représente chlore, brome, iode, alkylsulfonates (-OSO₂-alkyle, de préférence -OSO₂CH₃, -OSO₂CF₃) ou arylsulfonates (-OSO₂-aryle) ;
sont mis en réaction en un rapport molaire de 0,9 à 2,5, et, lors d'une deuxième étape B), 0 à 9 % en moles d'eau, par rapport à 1 mole du composé de formule (III), est ajoutée au mélange réactionnel, puis la séparation de la phase aqueuse du produit a lieu.

2. Procédé selon la revendication 1, **caractérisé en ce que** l'étape A) est réalisée avec exclusion d'un solvant organique.

3. Procédé selon la revendication 1, **caractérisé en ce que** les étapes A) et B) sont réalisées avec exclusion d'un solvant organique.

4. Procédé selon la revendication 1, **caractérisé en ce que** la température de réaction est comprise entre - 20 °C et 70 °C.

5. Procédé selon la revendication 1, **caractérisé en ce que** l'hydroxyde alcalin est l'hydroxyde de sodium.

6. Procédé selon la revendication 1, **caractérisé en ce qu'**une durée de post-réaction de 1 à 24 h suit l'ajout complet des composés de formule (II) et (III) à l'étape A).

7. Procédé selon la revendication 1, **caractérisé en ce que** l'étape B) est suivie par une étape de purification C), un solvant organique étant ajouté au mélange réactionnel en un rapport molaire entre le solvant et le produit brut de 1:1 à 1:50.

8. Procédé selon la revendication 7, **caractérisé en ce que** le milieu de réaction est ajusté à un pH (RT) compris entre 0 et 2 par ajout d'un acide minéral.

9. Procédé selon les revendications 7 et 8, **caractérisé en ce que** la séparation de la phase organique a lieu, la phase aqueuse étant mélangée avec un solvant organique et le rapport molaire entre le solvant et le produit brut étant de 10:1 à 1:50 et le pH (RT) étant ajusté entre 11 et 14 avec une base et la phase aqueuse étant séparée.

10. Procédé selon la revendication 1 ou 7, **caractérisé en ce que**, lors d'une étape D), l'élimination de résidus d'eau et éventuellement de résidus du solvant organique, ainsi que de résidus du composé de formule (II), a lieu par distillation.

11. Procédé selon la revendication 1, **caractérisé en ce qu'**il comprend les étapes A), B) et D).

12. Procédé selon la revendication 1, **caractérisé en ce qu'**il comprend les étapes A), B), C) et D).
